# EUROPEAN PATENT SPECIFICATION

(11) **EP 0 455 648 B1**
(45) Date of publication and mention of the grant of the patent: **10.03.1993**
(21) Application number: 90901388.0
(22) Date of filing: 18.12.1989
(51) Int. Cl.: A61K 9/22, A61K 31/44

(54) **DOSAGE FORM FOR ADMINISTERING CALCIUM ANTAGONIST**
DOSISFORM ZUR VERABREICHUNG VON CALCIUMANTAGONISTEN
FORME DE DOSAGE POUR ADMINISTRER UN ANTAGONISTE DU CALCIUM

(30) Priority: 30.01.1989 US 303706
(43) Date of publication of application: 13.11.1991
(73) Proprietor: ALZA CORPORATION, Palo Alto California 94303-0802 (US)
(72) Inventor: DETERS, Joseph, C., Napa, CA 94558 (US); SWANSON, David, R., Palo Alto, CA 94303 (US); DE ROSA, Stephen, C., Menlo Park, CA 94025 (US)
(74) Representative: Evans, David Charles
(86) International application number: US8905670
(87) International publication number: WO9008536

(56) References cited:
- EP-A- 0 238 189
- US-A- 4 519 801
- US-A- 4 783 337

## Description

### TECHNICAL FIELD

This invention pertains to a dosage form comprising the beneficial drug nicardipine useful for treating cardiovascular conditions. The invention also concerns a dosage form for use in a method for treating cardiovascular conditions by administering a dosage form that delivers nicardipine at a therapeutically effective rate for the management of the cardiovascular conditions.

### BACKGROUND OF THE INVENTION

The beneficial drug nicardipine, 1,4-dihydro-2,6-dimethyl-4-(3-nitrophenyl)-3,5-pyridinedicarboxylic acid methyl 2-methyl [(phenylmethyl)amino] ethyl ester and its pharmaceutically acceptable salts, is a calcium antagonist clinically useful for the treatment of cardiovascular conditions. Nicardipine is clinically useful for treating cardiovascular conditions such, as ischemia, hypertension, congestive heart failure, cerebrovascular diseases and coronary artery disease. Nicardipine reduces myocardial oxygen demand through coronary vasodilation and it has cardioprotective and vascular antispastic effects. Nicardipine's chemical structure and synthesis are disclosed in The Merck Index, 10th Ed., p 931, (1983). Nicardipine's therapeutic properties are disclosed in a study reported in Clinical Therapeutics, Vol. 10, pp 316 to 325, (1988). The drug was administered in the study intravenously for its cardiovascular effects.

In the light of the above presentation, it will be appreciated by the pharmaceutical and medical professions that a considerable need exists for an oral dosage form useful for administering nicardipine and its salts for the management of cardiovascular diseases and for its clinical relevance. The need exists for a dosage form that can deliver the valuable drug nicardipine and its salts at a rate controlled by the dosage form to a patient in critical need of nicardipine cardiovascular therapy. The pressing demand exists also for an oral dosage form that can deliver nicardipine at a controlled rate and at a constant does per unit time over a prolonged period of time for its beneficial hemodynamic effects, which delivery occurs substantially independent of the variable environment of the gastrointestinal tract. It will be appreciated further by those versed in the dispensing art, that such a novel and unique form that can administer nicardipine in a rate controlled dose over time, and simultaneously provide cardiovascular therapy, would represent an advancement and a valuable contribution to the arts.

Accordingly, in view of the above presentation, it is an immediate object of this invention to provide a dosage form for delivering nicardipine and its salts in a rate controlled amount, and which dosage form substantially overcomes the deficiencies associated with the prior art.

Another object of the present invention is to provide a dosage form for administering nicardipine in a rate controlled dose over a prolonged period of time for cardiovascular therapy.

Another object of the invention is to provide a pharmaceutical dosage form that makes available sustained and controlled nicardipine therapeutic activity.

Another object of the invention is to provide a novel dosage form manufactured as an osmotic device that can administer nicardipine to a biological receptor to produce the desired pharmaceutical effects.

Another object of the present invention is to provide a dosage form manufactured as an osmotic dosage form that substantially reduced and/or substantially eliminates the unwanted influences of the gastrointestinal environment of use and still provide controlled administration of nicardipine over time.

Another object of the present invention is to provide a dosage form for administering a member selected from the group consisting of nicardipine and its pharmaceutically acceptable salts for calcium antagonist therapy.

Another object of the present invention is to provide a dosage form adapted for oral administration of nicardipine, which dosage form comprises a first composition and a contacting second composition that act in harmony for the rate controlled administration of nicardipine over time.

Another object of the present invention is to provide a complete pharmaceutical regimen comprising a composition comprising nicardipine that can be dispensed from a drug delivery device, the use of which device requires intervention only for initiation and possibly for termination of the regimen.

Another object of the invention is to provide a dosage form for use in a method of treating cardiovascular diseases by orally administering nicardipine in a rate controlled dosage per unit time to a warm-blooded animal in need of cardiovascular therapy.

Other objects, features and advantages of the invention will be more apparent to those versed in the dispensing arts from the following detailed specification, taken in conjunction with the drawings and the accompanying claims.

### BRIEF DESCRIPTION OF THE DRAWINGS

In the drawing figures, which are not drawn to scale but are set forth to illustrate various embodiments of the invention, the drawing figures are as follows:
Figure 1 is a view of a dosage form designed and shaped for orally administering nicardipine or nicardipine and its salts to a gastrointestinal receptor of a warm-blooded animal;
Figure 2 is an opened view of the dosage form of Figure 1 for illustrating the structure of the dosage form;
Figure 3 is an opened view of the dosage form of Figure 1 depicting the dosage form manufactured comprising means for providing immediate drug delivery of nicardipine and means for providing controlled and prolonged drug delivery of nicardipine;
Figure 4 is an opened view of the dosage form of Figure 1 depicting a different structural embodiment of the dosage form provided by the invention; and,
Figures 5 through 12 depict release rates and cumulative amounts of nicardipine delivered over time by dosage forms provided by the invention.

### DETAILED DESCRIPTION OF THE INVENTION

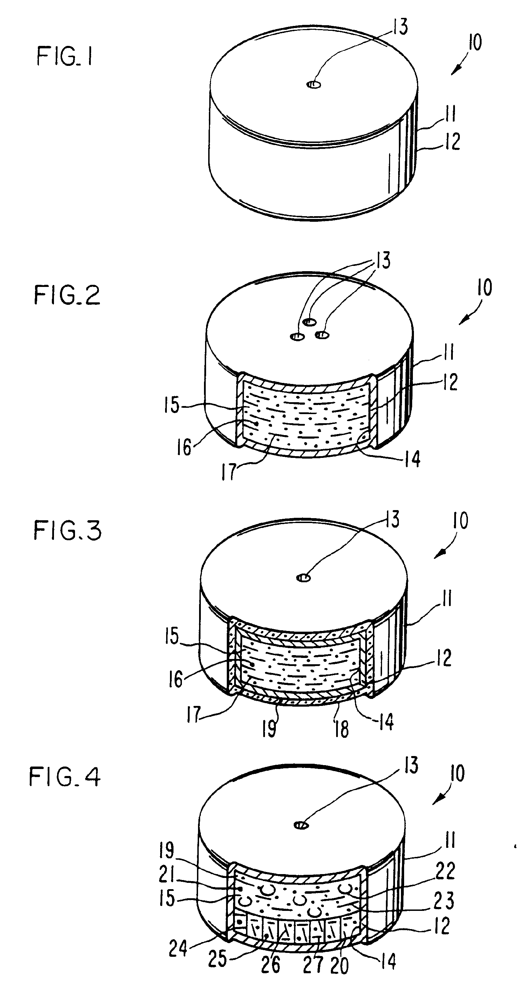

Turning now to the drawing Figures in detail, which drawing figures are an example of the dosage form provided by this invention, and which example is not to be construed as limiting, one example of the dosage form is illustrated in Figure 1 and designated by the numeral 10. In Figure 1, dosage form 10 comprises a body member 11 comprising a wall 12 that surrounds and encloses an internal compartment, not seen in Figure 1. Dosage form 10 comprises at least one exit means 13 for connecting the interior of dosage form 10 with the exterior environment of use.

In Figure 2, dosage form 10, manufactured as an osmotic device, is seen in opened view. In Figure 2, dosage form 10 comprises body 11, wall 12, that is sectioned at 14, and which wall surrounds and defines as internal compartment 15. Wall 12 comprises at least one exit means 13 that connects compartment 15 with the exterior of dosage form 10.

Wall 12 of dosage form 10 comprises in at least a part a composition that is permeable to the passage of an exterior fluid present in the environment of use. Wall 12 is substantially impermeable to the passage of nicardipine and ingredients present in compartment 15. Wall 12 comprises a composition that is substantially inert, and it maintains its physical and chemical integrity during the dispensing life of nicardipine from dosage form 10. The phrase keeps its physical and chemical integrity means wall 12 does not lose its structure and it does not change during the dispensing life of dosage form 10.

Wall 12, in one presently preferred embodiment comprises a cellulose ethyl ether, or wall 12 comprises a composition comprising a cellulose ethyl ether, a cellulose ether, and other wall forming members. More specifically, wall 12 comprises from 45 weight percent to 80 weight percent of ethylcellulose, from 5 weight percent to 30 weight percent hydroxypropylcellulose and from 5 weight percent to 30 weight percent polyethylene glycol, with the total weight percent of all components comprising wall 12 equal to 100 weight percent. In another specific embodiment, wall 12 comprises 45 weight percent to 80 weight percent of ethylcellulose, from 5 weight percent to 30 weight percent hydroxypropylcellulose, from 5 weight percent to 30 weight percent polyethylene glycol, and from 2 weight percent to 20 weight percent of polyvinyl pyrrolidone, with the total amount of all components comprising wall 12 equal to 100 weight percent.

Wall 12, in another presently preferred embodiment comprises 100 weight percent of a cellulose polymer comprising a member selected from the group consisting of a cellulose acylate, cellulose diacylate, cellulose triacylate, cellulose acetate, cellulose diacetate and cellulose triacetate. In another embodiment wall 12 comprises is a composition comprising from 60 weight percent to 95 weight percent of a member selected from the group consisting of a cellulose acylate, cellulose diacylate, cellulose triacylate, cellulose acetate, cellulose diacetate and cellulose triacetate, from 0 weight percent to 35 weight percent of a member selected from the group consisting of hydroxypropylcellulose and hydroxypropylmethylcellulose, and from 0 weight percent to 30 weight percent of polyethylene glycol, with the total amount of all components comprising wall 12 equal to 100 weight percent.

Internal compartment 15, in drawing figure 2, comprises from 5 mg to 150 mg of the therapeutic drug nicardipine, represented by dots 16. The drug nicardipine 16 can be present in the form of its pharmaceutically acceptable salts, such as those formed by hydrochloric acid, hydrobromic acid, sulfonic acid, phosphoric acid, acetic acid, propionic acid, citric acid, oxalic acid, maleic acid, chlorotheophylline, gluconic acid, and choline. Internal compartment 15 optionally comprises an osmagent 17, represented by dashes. The osmagents are known also as osmotically effective solutes. osmagents 17 operable for the present purpose comprise sodium chloride, potassium chloride, sodium sulfate, potassium sulfate, magnesium chloride, lithium chloride, potassium acid phosphate, tartaric acid, and raffinose. Generally compartment 15 optionally comprises from 1 mg to 75 mg of osmagent 17.

Figure 3 depicts another embodiment of dosage form 10, seen in opened section at 14. In drawing figure 3, dosage form 10 comprises body 11, wall 12, exit means 13, internal compartment 15, nicardipine 16 and osmotically effective compound 17. In Figure 3, dosage form 10 comprises additionally an overcoat 18 coated onto the exterior surface of wall 12. overcoat 18 comprises a composition comprising from 1 mg to 35 mg of nicardipine and its non-toxic salts, and an aqueous soluble carrier comprising hydroxypropylmethylcellulose. Overcoat 18 makes available instantly the drug nicardipine. In operation, when dosage form 10 is in a fluid environment of use, overcoat 18 dissolves or undergoes dissolution and concurrently delivers nicardipine 19 to the drug receptor.

In drawing Figure 4, another dosage form 10 provided by the invention is seen in opened section. In Figure 4, dosage form 10 comprises a body 11, wall 12, which wall 12 is sectioned at 14, with wall 12 surrounding and defining internal compartment 15. Wall 12 comprises at least one exit means 13 that connects compartment 15 with the exterior of dosage form 10. In dosage form 10, the internal compartment 15 comprises a first composition 19, which also can be defined optionally as a first lamina 19, and a second composition 20, which also can be defined optionally as a second lamina 20. First composition 19 and second composition 20 initially are in laminar arrangement and they cooperate with each other and with dosage form 10 for the effective delivery of nicardipine from dosage form 10.

In compartment 15, the first composition 19 comprises from 2 weight percent to 40 weight percent of nicardipine, or of nicardipine and its nontoxic salts, identified by dots 21; from 35 weight percent to 85 weight percent of a polyethylene oxide identified by curved lines 22, which polyethylene oxide 22 comprises a member selected from the group consisting of a polyethylene oxide comprising a molecular weight of about 100,000, a polyethylene oxide having a molecular weight of 200,000 a polyethylene oxide having a molecular weight of about 300,000, and a polyethylene oxide having a molecular weight of about 325,000; and from 0 weight percent to 20 weight percent of a hydroxypropylmethylcellulose having a number average molecular weight of 9,000 to 18,000, identified by dashes 23; which first composition 19 optionally comprises from zero weight percent to 3 weight percent of a lubricant, such as zero weight percent to 3 weight percent of magnesium stearate, with the total weight percent of all ingredients equal to 100 weight percent.

First composition 19, in more specified embodiments comprise, from 10 weight percent to 25 weight percent of nicardipine hydrochloride, from 75 weight percent to 80 weight percent of polyethylene comprising a 100,000 molecular weight, from 4 weight percent to 7.5 of hydroxypropylmethylcellulose comprising a 11,300 molecular weight and 0.3 weight percent to 0.75 weight percent magnesium stearate; and a first composition comprising from 35 weight percent to 45 weight percent nicardipine hydrochloride, from 40 weight percent to 50 weight percent of a polyethylene oxide comprising a 200,000 molecular weight, from 5 weight percent to 15 weight percent of a polyethylene oxide comprising a 300,000 molecular weight, from 3 weight percent to 8 weight percent of hydroxypropylmethylcellulose comprising a 11,300 molecular weight, and from 0.3 weight percent to 0.75 weight percent of magnesium stearate.

The second composition 20 comprises form 50 weight percent to 75 weight percent of a polyethylene oxide comprising a 4,500,000 to 5,500,000 molecular weight identified by vertical lines 24; from 15 weight percent to 35 weight percent of an osmagent identified by dots 25; from 3 weight percent to 15 weight percent of a hydroxypropylmethylcellulose comprising a 9,000 to 18,000 molecular weight identified by slanted lines 26; from zero weight percent to 3 weight percent of a lubricant, such as stearic acid, or magnesium stearate, identified by short dashes 27; and from zero weight percent to 3 weight percent of a colorant, such as ferric oxide, with the total weight percent of all ingredients equal to 100 weight percent.

The second composition 20, in more specific embodiments comprises 60 weight percent to 70 weight percent of a polyethylene oxide comprising a 5,000,000 molecular weight, from 25 weight percent to 35 weight percent of osmagent sodium chloride, from 4 weight percent to 6 weight percent of a hydroxypropylmethylcellulose comprising a 11,300 molecular weight, from 0.75 weight percent to 1.25 weight percent ferric oxide, and from 0.4 weight percent to 0.7 weight percent magnesium stearate. The presence of the polyethylene oxide and the osmagent in the second composition increases the operating efficiency of dosage form 10. The operating efficiency occurs by the simultaneous hydration and swelling of the polyethylene oxide coupled with the osmotic imbibition of exterior fluid through the semipermeable wall by the osmagent at a rate dependent on the concentration gradient across the wall. These combined physical actions are maintained at a high level over a prolonged period of time, thereby enabling the second compositions to push the first composition at a more constant and uniform rate over a corresponding prolonged period of time. The constant push against the first compartment assures a more uniform rate of release of nicardipine from the dosage form and concomitantly substantially prevents a declining and decreasing release rate over time. The polyethylene oxides used for manufacturing dosage form 10 is commercially available from the Union Carbide Corporation, South Charleston, Hest Virginia.

The expression, "exit means 13," as used herein, comprises means and methods suitable for the metered release of the beneficial drug nicardipine and/or its salts from dosage form 10. The means 13 includes a passageway or orifice, through wall 12 for communicating with nicardipine in dosage form 10. The expression, "passageway," includes aperture, orifice, bore, pore, porous element through which the drug can migrate, hollow fiber, capillary tube, porous overlay, and porous insert. The expression also includes a material that erodes or is leached from wall 12 in the fluid environment of use to produce one passageway in dosage form 10. Representative materials suitable for forming one passageway, or a multiplicity of passageways, include an erodible poly(glycolic) acid or poly(lactic) acid member in the wall; a gelatinous filament; poly(vinyl alcohol); leachable materials such as a removable pore forming polysaccharide, salt or oxide. A passageway, or a plurality of passageways can be formed by leaching a material such as sorbitol, lactose, or maltose from the wall. The passageway can have any shape such as round, triangular, square, elliptical, or other irregular shapes, for assisting in the metered release of nicardipine from dosage form 10. Dosage form 10 can be constructed with one, or more passageways in spaced apart relations, or more than a single surface of a dosage form. Passageways are disclosed in United States Patents Nos. 3,845,770; 3,916,899; 4,063,064; and 4,088,864. Passageways formed by leaching are disclosed in United States Patent Nos. 4,200,098, and 4,285,987.

The osmagents used for the purpose of the present invention are also known as osmotically effective compounds and as osmotically effective solutes. The osmagents exhibit an osmotic pressure gradient across a semipermeable wall against an external fluid. The osmagent, along with the osmopolymer, imbibe fluid into the dosage form, thereby making available in situ fluid for imbibition and/or absorption by an osmopolymer to enhance its expansion for pushing the beneficial drug nicardipine from the dosage form. The osmagents can be inorganic or organic. osmagents useful for the present purpose include magnesium sulfate, magnesium chloride, potassium sulfate, sodium sulfate, lithium sulfate, potassium acid phosphate, mannitol, sodium chloride, potassium chloride, urea, inositol, tartaric acid, raffinose, sucrose, glucose, sorbitol, and mixtures thereof. osmagents are known to the prior art in U:S. Pat. No. 4,783,337.

The dosage form of the invention is manufactured by standard techniques. For example, in one embodiment the beneficial drug nicardipine is formulated into a first composition comprising from 5 mg to 150 mg of a member selected from the group consisting of nicardipine and its pharmaceutically acceptable salts, a polyethylene oxide having a 100,000 to 325,000 molecular weight, and a hydroxypropylmethylcellulose having a molecular weight of 9,000 to 18,000; or into a first composition comprising from 5 mg to 150 mg of nicardipine and its salts; a pair of polyethylene oxides comprising a 100,000 and 200,000 molecular weight, or a pair of polyethylene oxides comprising a 200,000 and a 300,000 molecular weight; and a hydroxypropylmethylcellulose comprising a molecular weight of 9,000 to 18,000; by mixing nicardipine or its salts with the compositional forming ingredients and pressed into a lamina possessing dimensions that correspond to the internal dimensions of the space adjacent to the passageway of the dosage form. In another manufacturing embodiment, the beneficial drug nicardipine and other first composition forming ingredients and a solvent are mixed into a solid, or a semisolid by conventional methods such as ballmilling, calendering, stirring, or rollmilling, and then pressed into a preselected lamina forming shape. Next, a limina, or a second composition comprising an osmopolymer and an osmagent, such as a second composition comprising a polyethylene oxide having a molecular weight greater that 4,500,000 and a hydroxypropylmethylcellulose possessing a 9,000 to 18,000 molecular weight, or a second composition comprising a polyethylene oxide having a molecular weight greater than 4,500,000 and a hydroxypropylmethylcellulose possessing a 9,000 to 18,000 molecular weight are placed in contact with the lamina comprising the beneficial nicardipine, and the two lamina comprising the laminates are surrounded with a wall. The lamination of the first beneficial composition comprising the nicardipine and the second composition comprising the osmopolymer and the osmagent can be accomplished by using a conventional two-layered tablet press. The wall can be applied by molding, spraying, or dipping the pressed shapes into wall forming materials. Another and presently preferred technique that can be used for applying the wall is the air suspension coating procedure. This procedure consists in suspending and tumbling the two-layered laminate in a current of air until the wall forming composition surrounds the laminate. The air suspension procedure is described in U.S. Pat. No. 2,799,241; in J. Am. Pharm. Assoc., Vol. 48, pp 451-459 (1979); and, ibid, Vol. 49, PP 82-84 (1960). Other standard manufacturing procedures are described in Modern Plastic Encyclopedia, Vol. 46, pp 62-70 (1969); and in Pharmaceutical Sciences, by Remington, 14th Ed., pp 1626-1978, (1970), published by Mack Publishing Co., Easton, PA.

Exemplary solvents suitable for manufacturing the wall, the laminates and laminae include inert inorganic and organic solvents that do not adversely harm the materials, and the final wall. The solvents broadly include a member selected from the group consisting of aqueous, alcohol, ketone, ester, ether, aliphatic hydrocarbon, halogenate, cycloaliphatic, aromatic heterocyclic solvents and mixtures thereof. Typical solvents include acetone, diacetone, alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethyl acetate, methylene dichloride, ethylene dichloride, propylene dichloride, carbon tetrachloride, chloroform, nitroethane, nitropropane. tetrachloroethane, ethyl ether, isopropyl ether, cyclohexane, cyclooctane, benzene, toluene, naphtha, 1,4-dioxane, tetrahydrofuran, diglyme, aqueous and nonaqueous mixtures, acetone and water, acetone and methanol, acetone and ethyl alcohol, methylene dichloride and methanol, ethylene dichloride and methanol, ethyl alcohol and water.

### DETAILED DESCRIPTION OF EXAMPLES

The following examples are merely illustrative of the present invention and they should not be considered as limiting the scope of the invention in any way, as these examples and other equivalents thereof will become more apparent to those versed in the art in the light of the present disclosure, the drawings and the accompanying claims.

### EXAMPLE 1

A dosage form adapted, designed and shaped as an osmotic drug delivery system was manufactured as follows: first, 1.92 kg of nicardipine hydrochloride was dry blended with 7.54 kg of polyethylene oxide comprising a 100,000 molecular weight, 1.88 kg of polyethylene oxide comprising a 200,000 molecular weight and 0.60 kg of hydroxypropylmethylcellulose comprising a 11,200 molecular weight for 15 minutes in a Hobart® mixer. All the materials were pre-screened to 30 mesh. Next, 7.5 liters of anhydrous ethyl alcohol was added to the blended materials, followed by 15 minutes of additional blending in the blender. Then, the wet mass was passed through a stainless steel screen with 1/4 inch openings, about 6 mm, using a Fitzmill® comminutor, at low speed. Next, the screened blend was dried in an oven at 70°F, about 22°C, for 20 hours. The dried granules were passed through a 14 mesh screen, and then 56 g of magnesium stearate were added to yield 11,112 g of drug nicardipine granules.

In a separate operation, 16.125 kg of polyethylene coagulant comprising a 5,000,000 molecular weight was mixed with 7.250 kg of sodium chloride, 1.25 kg of hydroxypropylmethylcellulose comprising a 11,200 molecular weight and 0.25 kg of red iron oxide. The sodium chloride and the iron oxide were pre-screened through a 20 mesh screen. Then, 18 l of anhydrous ethyl alcohol was added with mixing, followed by 15 minutes of additional mixing to produce a uniform blend. The wet mass was passed through a stainless steel screen with 6 mm opening using a Fluid Air® mill at 500 rpm. Then, the granules were dried in a steam-heated oven at 22°C for 70 hrs. The dried granules were passed through a 10 mesh screen using a fluid air mill operating at 1500 rpm. Next, 120 g of magnesium stearate was added with blending to yield 24.02 kg, of osmotic driving composition.

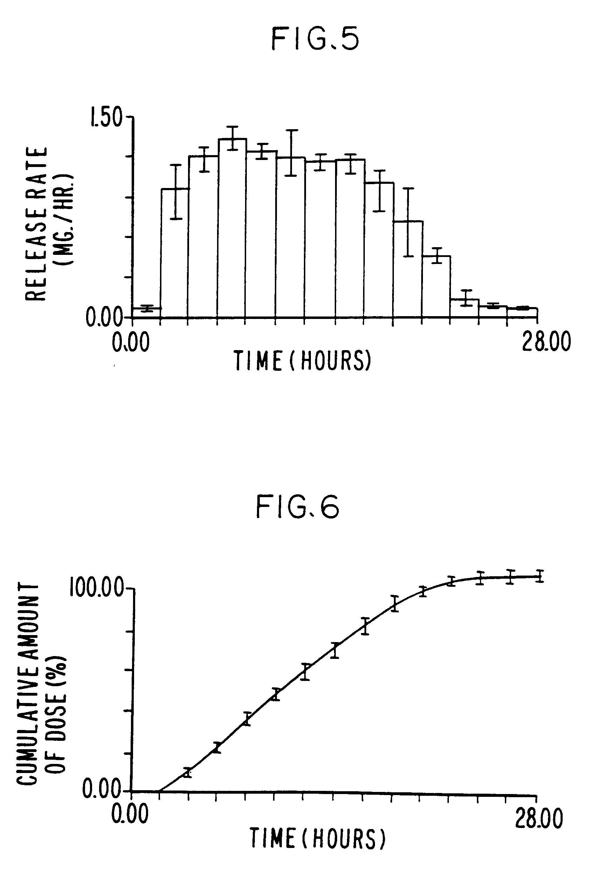

Next, the nicardipine drug granules and the osmotic driving composition were loaded into the hopper of a Kilian® tablet press comprising a bilayer mode. Then, 12,857 tablets were compressed at an average weight of 239.2 mg each. The compression speed was 21 rpm. The compressed, bilayer cores exhibited a hardness of 7-8 kp, and they were 11/32 inches, about 10 mm, in diameter. Then, 1000 of the cores were placed in a 6-inch, about 15 cm, truncated column and coated using a standard air suspension technique. The wall coating composition comprised 94% cellulose acetate comprising a 39.8% acetyl content and 6% polyethylene glycol 3350 dissolved in a 4% (weight/weight) solution in 90% methylene chloride/10% methanol (w/w). About 3,600 g of coating solution was applied to the cores. The wet weight gain for each core was 35.7 mg. A 25 mil, 0.635 mm, passageway was laser drilled in each coated core. Then, the dosage forms were dried at 45° C and 45% relative humidity for 70 hrs., followed by 3 hrs. at 45° C without humidity. The dry membrane weight is about 30 mg. The final dosage forms comprised 21.10 mg of nicardipine hydrochloride. The release rate in mg/hr. and the cumulative amount of nicardipine released over a prolonged period of 28 hrs. is seen in accompanying Fig. 5 and Fig. 6.

### EXAMPLE 2

The procedure set forth in Example 1 was followed in this example. The composition comprising the drug nicardipine used in this example was prepared according to Example 1. The osmotic driving composition was prepared as described, and in this example the osmotic driving composition comprises 64.5% polyethylene oxide Coagulant® possessing a 5,000,000 molecular weight, 29% of sodium chloride, 5% of hydroxypropylmethylcellulose possessing a 11,200 molecular weight, 1% of iron oxide, and 0.5% of magnesium stearate. The drug nicardipine composition and the osmotic driving composition were compressed under 2.25 tons of compression force into bilayer cores.

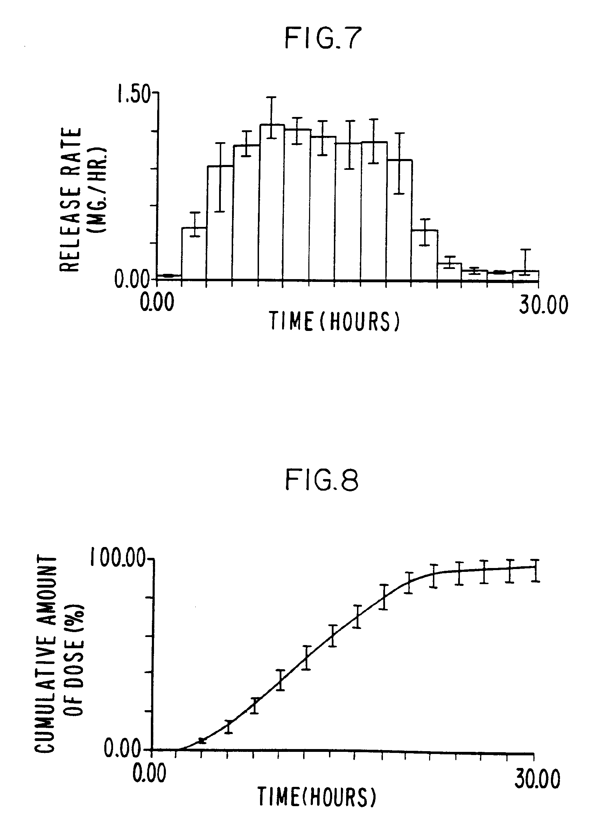

Next, 1,000 g of the bilayer cores were coated in a Wurster® air suspension coater. The coating solution comprised 241.4 g of ethyl cellulose, 62.2 g of hydroxypropylcellulose and 62.2 g of polyethylene glycol all dissolved in 10,000 ml of anhydrous ethyl alcohol and 877.8 ml of sterile water. The bilayers were coated at a flow rate of 38-40 ml/min. until the bilayers were uniformly coated with the wall forming composition. The average coat applied to each bilayer was about 37.1 mg. The coated bilayers were laser drilled to effect a 24 mil, 0.609 mm, passageway in each drug delivery system. The delivery systems-were dried for 72 hrs. at 50° C and the average dry wall weighed about 33.5 mg. Each drug delivery system comprised 22 mg of nicardipine hydrochloride. The release rate in mg/hr., and the cumulative amount released over an extended period of time are seen in accompanying Fig. 7 and Fig. 8.

### EXAMPLE 3

The procedure of Examples 1 and 2 was followed in this example, with the conditions as previously set forth, except that in the present example, the wall forming composition comprises 280.9 g of ethyl cellulose, 79 g of hydroxypropylcellulose, 79 g of polyethylene oxide, 12,000 ml of anhydrous ethyl alcohol and 1.053 l of sterile water. The dosage form comprised a dry wall of 44.8 mg and a passageway of 23.6 mil, 0.599 mm.

### EXAMPLE 4

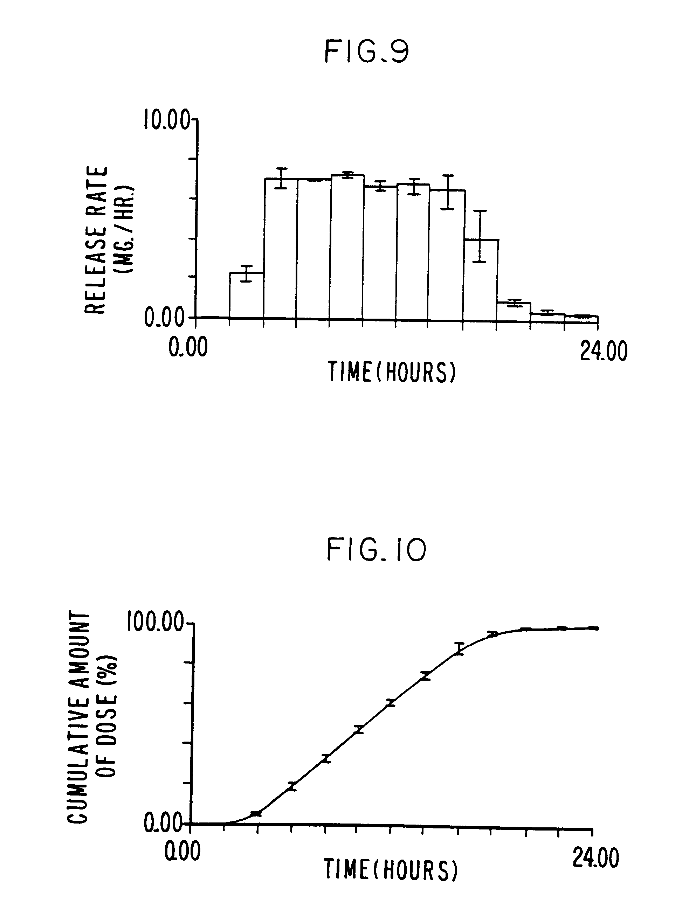

The procedure of Examples 1 and 2 were followed in this example, with all procedures as previously described, except that in this example the drug layer comprises 40 wt % nicardipine hydrochloride, 46.33 wt % polyethylene oxide with a 200,000 molecular weight, 8.18 wt % polyethylene oxide, having a 300,000 molecular weight, 5 wt % hydroxypropylmethylcellulose having a 11,200 molecular weight, and 0.50 wt % of magnesium stearate. The osmotic driving composition comprised 64.50 wt % polyethylene oxide coagulant possessing a 5,000,000 molecular weight, 29 wt % of sodium chloride, 5 wt % hydroxypropylmethylcellulose possessing a 11,200 molecular weight, 1 wt % ferric oxide, and 0.5 wt % magnesium stearate. The wall of the dosage form comprises 60 wt % ethyl cellulose, 20 wt % hydroxypropylcellulose and 20 wt % polyethylene glycol. The dosage form comprises a 0.76 mm orifice, and exhibited a rate of release and cumulative amount release as seen in Fig. 9 and Fig. 10.

### EXAMPLE 5

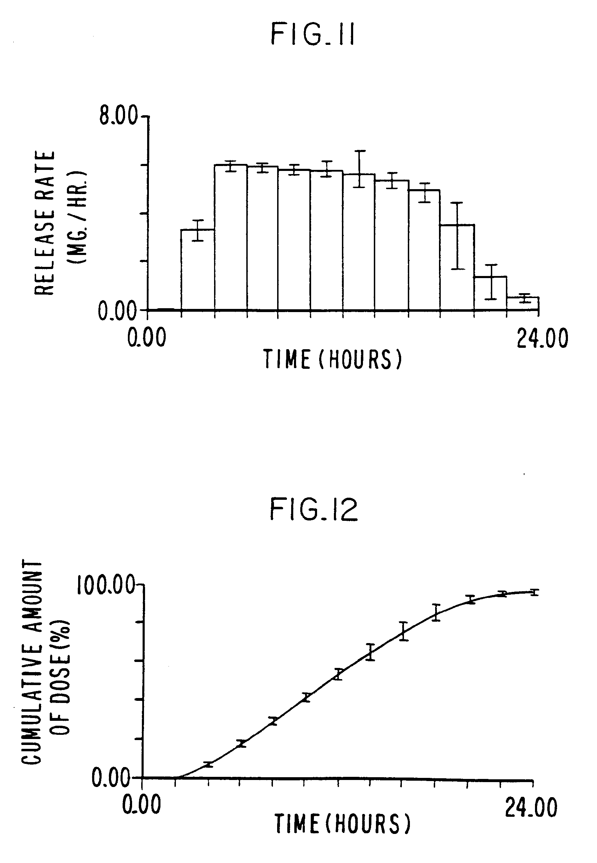

The procedures set forth herein were followed in this example, wherein the drug layer comprises 110 mg of nicardipine hydrochloride, 127.4 mg of polyethylene oxide having a 200,000 molecular weight, 22.5 mg of polyethylene oxide having a 300,000 molecular weight, 13.7 mg of hydroxypropylmethylcellulose having a 11,200 molecular weight, and 1.4 mg of magnesium stearate. The osmotic driving composition comprises 118.7 of polyethylene oxide having a molecular weight of 5,000,000, 53.4 mg of sodium chloride, 9.2 mg of hydroxypropylmethylcellulose having a 11,200 molecular weight, 1.8 mg of ferric oxide, and 0.9 mg of magnesium stearate. The wall of the dosage form comprises 45.1 mg of cellulose acetate having a 39.8% acetyl content and 5 mg of polyethylene glycol. The dosage form comprises a 0.79 mm orifice and exhibits a release rate and a cumulative amount release over time curves as seen in Fig. 11 and Fig. 12.

### EXAMPLE 6

A series of dosage forms are prepared for delivery of nicardipine at a controlled rate orally to a warm-blooded animal in need of nicardipine therapy. The dosage forms are administered according to the method comprising the steps of: (A) admitting into a warm-blooded animal a dosage form comprising: (1) a wall surrounding a compartment, the wall selected from the group consisting of a composition comprising 20 mg to 30 mg of ethyl cellulose, 4 mg to 10 mg of hydroxypropylcellulose and 4 mg to 10 mg of polyethylene glycol; or, a composition comprising 30 mg to 40 mg of ethyl cellulose, 10 mg to 15 mg of hydroxypropylcellulose and 10 mg to 15 mg of polyethylene glycol; or, a composition comprising 15 mg to 25 mg of a cellulose acetate and 0.5 mg to 2 mg of polyethylene glycol; or, a composition comprising 40 mg to 50 mg of cellulose acetate and 3 mg to 8 mg of polyethylene glycol; (2) a compositional layer in the compartment comprising nicardipine present in an amount for performing a therapeutic program, said composition comprising 15 mg to 30 mg of nicardipine, from 80 mg to 90 mg of polyethylene oxide having a 200,000 molecular weight, from 15 mg to 25 mg of a polyethylene oxide having a 300,000 molecular weight, from 5 mg to 8 mg of hydroxypropylmethylcellulose, and from 0.5 mg to 1.5 mg of polyethylene glycol; or, a compositional layer in the compartment comprising nicardipine present in an amount for performing a therapeutic program, said composition comprising from 105 to 115 mg of nicardipine, from 120 mg to 130 mg of a polyethylene oxide having a 200,000 molecular weight, from 18 mg to 28 mg of a polyethylene oxide having a 300,000 molecular weight, from 10 mg to 15 mg of a hydroxypropylmethylcellulose having a 11,200 molecular weight, and layer in the compartment for pushing the nicardipine composition from the dosage form, said pushing composition comprising from 60 mg to 70 mg of a polyethylene oxide Coagulant having a 5,000,000 molecular weight, from 25 mg to 35 mg of sodium chloride, from 3 mg to 8 mg of hydroxypropylmethylcellulose having a 11,200 molecular weight, from 0.5 mg to 1.5 mg of ferric oxide and from 0.2 mg to 0.75 of magnesium stearate; or a push composition comprising 110 mg to 125 mg of polyethylene oxide Coagulant having a 5,000,000 molecular weight, from 50 mg to 55 mg of sodium chloride, from 6 mg to 12 mg of hydroxypropylmethylcellulose having a 11,200 molecular weight, from 1 mg to 3 mg of ferric oxide and from 0.7 mg to 1.5 mg of magnesium stearate; and (4) one passageway in the wall for releasing the nicardipine; (B) imbibing fluid through the semipermeable part of the wall and the osmotic pressure gradient across the semipermeable wall causing the drug layer composition to hydrate and reduce viscosity and the osmotic push composition to expand and swell; and (C) deliver the beneficial nicardipine from the dosage form through the exit passageway to the warm blooded animal over a prolonged period of time.

## Claims

1. A dosage form for administering nicardipine to a patient, wherein the dosage form comprises:
(a) a wall comprising from 15 weight percent to 80 weight percent of ethylcellulose, from 5 weight percent to 30 weight percent hydroxypropylcellulose, and from 5 weight percent to 30 weight percent of polyethylene glycol, which wall surrounds:
(b) a compartment comprising a bilayer core, which core comprises:
(c) a first composition from 5 mg to 150 mg of a member selected from the group consisting of nicardipine and its pharmaceutically acceptable salts, a polyethylene oxide having a 100,000 to 325,000 molecular weight, and a
hydroxypropylmethylcellulose having a molecular weight of 9,000 to 18,000;
(d) a second contacting composition comprising a polyethylene oxide having a molecular weight greater than 4,500,000 and a hydroxypropylmethylcellulose possessing a 9,000 to 18,000 molecular weight in the compartment that acts in harmony with the first composition for pushing the first composition from the dosage form for rate controled administration of nicardipine over time; and,
(e) at least one exit passageway in the wall for delivering nicardipine to the patient.

2. A dosage form for administering nicardipine to a patient, wherein the dosage form comprises:
(a) a wall comprising from 60 weight percent to 95 weight percent of cellulose acylate, from 0 weight percent to 35 weight percent of hydroxypropylcellulose, and from 0 weight percent to 30 weight percent of polyethylene glycol, with the total of all components equal to 100 weight percent, which wall surrounds:
(b) a compartment comprising a bilayer core, which core comprises:
(c) a first composition in the compartment comprising from 5 mg to 150 mg of nicardipine and its salts; a pair of polyethylene oxide comprising a 100,000 and 200,000 molecular weight, or a pair of polyethylene oxides comprising a 200,000 and a 300,000 molecular weight; and a hydroxypropylmethylcellulose comprising molecular weight of 9,000 to 18,000;
(d) a second composition in contact with the first composition, which second composition comprises a polyethylene oxide having a molecular weight greater than 4,500,000 and a hydroxypropylmethylcellulose possessing a 9,000 to 18,000 molecular weight in the compartment which second composition pushes the first composition comprising the nicardipine from the dosage form; and,
(e) at least one exit passageway in the wall for delivering the nicardipine from the dosage form at a controlled rate over time.

## Patentansprüche

1. Dosisform zur Verabreichung von Nicardipin an einen Patienten, wobei die Dosisform folgende Bestandteile umfaßt:
(a) eine 45 Gew.-% bis 80 Gew.-% Ethylcellulose, 5 Gew.-% bis 30 Gew.-% Hydroxypropylcellulose und 5 Gew.-% bis 30 Gew.-% Polyethylenglykol umfassende Wandung, wobei die Wandung umgibt
(b) eine Kammer, die einen aus zwei Schichten bestehenden Kern umfaßt, wobei der Kern folgende Bestandteile umfaßt:
(c) eine erste Zubereitung, die 5 mg bis 150 mg einer Verbindung, die aus der aus Nicardipin und seinen pharmazeutisch annehmbaren Salzen bestehenden Gruppe gewählt ist, ein Polyethylenoxid mit einem Molekulargewicht von 100.000 bis 325.000 und eine Hydroxypropylmethylcellulose mit einem Molekulargewicht von 9.000 bis 18.000 umfaßt;
(d) eine zweite, in Kontakt damit stehende Zubereitung, die ein Polyethylenoxid mit einem Molekulargewicht größer als 4.500.000 und eine Hydroxypropylmethylcellulose mit einem Molekulargewicht von 9.000 bis 18.000 in der Kammer umfaßt, wobei die zweite Zubereitung zusammen mit der ersten Zubereitung in der Weise wirkt, daß sie die erste Zubereitung zur geschwindigkeitsgeregelten Verabreichung von Nicardipin über die Zeit aus der Dosisform herausdrückt; und
(e) wenigstens eine Durchtrittsöffnung in der Wandung zur Abgabe von Nicardipin an den Patienten.

2. Dosisform zur Verabreichung von Nicardipin an einen Patienten, wobei die Dosisform folgende Bestandteile umfaßt:
(a) eine 60 Gew.-% bis 95 Gew.-% Celluloseacylat, 0 Gew.-% bis 35 Gew.-% Hydroxypropylcellulose und 0 Gew.-% bis 30 Gew.-% Polyethylenglykol umfassende Wandung, wobei die Gesamtmenge aller Komponenten gleich 100 Gew.-% ist, wobei die Wandung umgibt
(b) eine Kammer, die einen aus zwei Schichten bestehenden Kern umfaßt, wobei der Kern folgende Bestandteile umfaßt:
(c) eine erste Zubereitung in der Kammer, die 5 mg bis 150 mg Nicardipin und seine Salze, ein aus Polyethylenoxid mit einem Molekulargewicht von 100.000 und von 200.000 bestehendes Verbindungspaar oder ein aus Polyethylenoxid mit einem Molekulargewicht von 200.000 und von 300.000 bestehendes Verbindungspaar und eine Hydroxypropylmethylcellulose mit einem Molekulargewicht von 9.000 bis 18.000 umfaßt;
(d) eine zweite Zubereitung, die in Kontakt mit der ersten Zubereitung steht, wobei die zweite Zubereitung ein Polyethylenoxid mit einem Molekulargewicht größer als 4.500.000 und eine Hydroxypropylmethylcellulose mit einem Molekulargewicht von 9.000 bis 18.000 in der Kammer umfaßt, wobei die zweite Zubereitung die erste Zubereitung, die das Nicardipin umfaßt, aus der Dosisform herausdrückt; und
(e) wenigstens eine Durchtrittsöffnung in der Wandung zur Abgabe von Nicardipin aus der Dosisform mit einer über die Zeit geregelten Geschwindigkeit.

## Revendications

1. Forme posologique pour administrer la nicardipine à un malade, le forme posologique comprenant:
(a) une paroi comprenant de 45% en poids à 80% en poids d'éthylcellulose, de 5% en poids à 30% en poids d'hydroxypropylcellulose, et de 5% en poids à 30% en poids de polyéthylène glycol, laquelle paroi entoure:
(b) un compartiment comprenant un noyau bicouche, lequel noyau comprend:
(c) une première composition comprenant de 5 mg à 150 mg d'un élément choisi dans le groupe constitué par la nicardipine et ses sels pharmaceutiquement acceptables, un oxyde de polyéthylène ayant un poids moléculaire de 100 000 à 325 000, et une hydroxypropylméthylcellulose ayant un poids moléculaire de 9000 à 18 000;
(d) une seconde composition de contact comprenant un oxyde de polyéthylène ayant un poids moléculaire supérieur à 4 500 000 et une hydroxypropylméthylcellulose possédant un poids moléculaire de 9000 à 18 000 dans le compartiment qui agit en harmonie avec la première composition pour pousser la première composition à partir de la forme posologique en vue d'une administration contrôlée de la nicardipine avec le temps; et
(e) au moins un passage de sortie dans la paroi pour délivrer la nicardipine au malade.

2. Forme posologique pour administrer la nicardipine à un malade, comprenant:
(a) une paroi comprenant de 60% en poids à 95% en poids d'acylate de cellulose, de 0% en poids à 35% en poids d'hydroxypropylcellulose, et de 0% en poids à 30% en poids de polyéthylène glycol, le total de tous les composants étant égal à 100% en poids, laquelle paroi entoure:
(b) un compartiment comprenant un noyau bicouche, lequel noyau comprend:
(c) une première composition dans le compartiment comprenant de 5 mg à 150 mg de nicardipine et de ses sels; une paire d'oxydes de polyéthylène de poids moléculaires 100 000 et 200 000, ou une paire d'oxydes de polyéthylène de poids moléculaires 200 000 et 300 000; et une hydroxypropylméthylcellulose de poids moléculaires allant de 9000 à 18 000;
(d) une seconde composition au contact avec la première composition, laquelle seconde composition comprend un oxyde de polyéthylène ayant un poids moléculaire supérieur à 4 500 000 et une hydroxypropylméthylcellulose ayant un poids moléculaire de 9000 à 18 000 dans le compartiment, ladite seconde composition pousse la première composition comprenant la nicardipine hors de la forme posologique; et
(e) au moins un passage de sortie dans la paroi pour fournir la nicardipine à partir de la forme posologique à une vitesse contrôlée avec le temps.
